# EUROPEAN PATENT SPECIFICATION

(11) **EP 4 083 012 B1**
(45) Date of publication and mention of the grant of the patent: **25.12.2024**
(21) Application number: 20906967.3
(22) Date of filing: 15.12.2020
(51) Int. Cl.: C07C 209/72, C07C 211/18, C07B 61/00

(54) **METHOD FOR MANUFACTURING BIS(AMINOMETHYL)CYCLOHEXANE**
VERFAHREN ZUR HERSTELLUNG VON BIS(AMINOMETHYL)CYCLOHEXAN
PROCÉDÉ DE FABRICATION DE BIS(AMINOMÉTHYL)CYCLOHEXANE

(30) Priority: 27.12.2019 JP 2019239639
(43) Date of publication of application: 02.11.2022
(73) Proprietor: MITSUBISHI GAS CHEMICAL COMPANY, INC., Chiyoda-ku Tokyo 100-8324 (JP)
(72) Inventor: NOGUCHI, Emi, Kurashiki-shi, Okayama 712-8525 (JP); IBI, Yukiya, Kurashiki-shi, Okayama 712-8525 (JP); KUMANO, Tatsuyuki, Kurashiki-shi, Okayama 712-8525 (JP)
(74) Representative: Hoffmann Eitle
(86) International application number: PCT/JP2020/046785
(87) International publication number: WO 2021/131912

(56) References cited:
- DE-A1- 2 511 280
- JP-A- 2004 107 327
- JP-A- 2004 107 327
- JP-A- H08 143 514
- JP-A- S5 174 997
- JP-A- S50 126 638
- JP-A- S55 104 236
- US-A- 5 741 928

## Description

### Technical Field

The present invention relates to a method for producing bis(aminomethyl)cyclohexane.

### Background Art

Bis(aminomethyl)cyclohexane is an industrially important compound as the starting material of bis(isocyanatomethyl)cyclohexane or the like. Bis(aminomethyl)cyclohexane can be obtained, for example, by the catalytic hydrogenation treatment of xylylenediamine. PTL1 describes a method for producing bis(aminomethyl)cyclohexane by catalytic hydrogenation using a ruthenium catalyst and a solvent selected from the group consisting of alkylamines and alkylenediamines.

PTL2 relates to the regeneration treatment of a catalyst used for the hydrogenation of dicyanobenzene, which is an aromatic dinitrile. Specifically, it is disclosed that a catalyst (nickel catalyst) used in a liquid phase for the hydrogenation of dicyanobenzene can be regenerated by bringing the catalyst into contact with a hydrogen-containing gas in the temperature range of 200 to 500°C.

### Citation List

### Patent Literature

PTL1: JP 3974198 B
PTL2: JP 2004-107327 A
PTL3: US 5 741 928 A

### Summary of Invention

### Technical Problem

Also in the production of bis(aminomethyl)cyclohexane, as in PTL2, a problem is that the catalytic activity decreases gradually with use, and a satisfactory yield is not obtained.

But a problem has been that even if a catalyst used for the hydrogenation of xylylenediamine is treated by the regeneration method disclosed in PTL2, catalyst regeneration cannot be sufficiently performed. This problem has been studied, and it has been found that the following problem is caused by a deposit that forms when an attempt is made to regenerate a catalyst used for the hydrogenation of xylylenediamine with a hydrogen-containing gas. For example, in a reaction apparatus in a fixed-bed form, a deposit causes malfunctions in a heat exchanger, a filter, and the like on a production line, and the continuation of operation is impossible. Further, a deposit may form on the catalyst, and another problem is that the deposit inhibits catalyst regeneration. PTL1 does not describe the disclosure of the regeneration of the catalyst used for the reaction.

It is an object of the present invention to provide a method for producing bis(aminomethyl)cyclohexane in which in the production of bis(aminomethyl)cyclohexane by the hydrogenation reaction of xylylenediamine, a catalyst with decreased activity due to the reaction can be regenerated and reused as a catalyst.

### Solution to Problem

As a result of diligent studies, the present inventors have found that the problems can be solved by maintaining the amount of bis(aminomethyl)cyclohexane in a liquid before the step of contact with a hydrogen-containing gas at a particular amount in catalyst regeneration with a hydrogen-containing gas. That is, the present invention relates to the following.

A method for producing bis(aminomethyl)cyclohexane, including hydrogenating xylylenediamine in the presence of a solvent and a catalyst in a fixed-bed continuous flow type reactor, wherein the catalyst with decreased activity due to use is treated in said reactor in a catalyst regeneration treatment step including the following step (1) and step (2), and then reused in a reaction system:
step (1): maintaining an amount of bis(aminomethyl)cyclohexane in a liquid before the step (2) at 20% by mass or less,
step (2): heating the catalyst to 100 to 500°C and bringing the catalyst into contact with a hydrogen-containing gas.

### Advantageous Effects of Invention

According to the present invention, it is possible to provide a method for producing bis(aminomethyl)cyclohexane in which also in the production of bis(aminomethyl)cyclohexane by the hydrogenation of xylylenediamine, a catalyst with decreased activity due to use can be subjected to regeneration treatment. According to the production method of the present invention, catalytic ability is recovered to allow reuse, and the deposition of solid components in the production apparatus during catalyst regeneration, and the like can be prevented. Therefore, apparatus uncontrollability and catalyst deterioration during production can be avoided, and bis(aminomethyl)cyclohexane can be safely and efficiently produced.

### Description of Embodiment

The present invention relates to a method for producing bis(aminomethyl)cyclohexane, including hydrogenating xylylenediamine in the presence of a solvent and a catalyst in a fixed-bed continuous flow type reactor, wherein the catalyst with decreased activity due to use is treated in said reactor in a catalyst regeneration treatment step including the following step (1) and step (2), and then reused in a reaction system:
step (1): maintaining an amount of bis(aminomethyl)cyclohexane in a liquid before the step (2) at 20% by mass or less,
step (2): heating the catalyst to 100 to 500°C and bringing the catalyst into contact with a hydrogen-containing gas.

A mode for carrying out the present invention (hereinafter simply referred to as "this embodiment") will be described in detail below. This embodiment is an illustration for describing the present invention and is not intended to limit the present invention to the following contents. Appropriate modifications can be made to the present invention without departing from the scope of the claims. As used herein, the description "XX to YY" means "XX or more and YY or less".

### <Production of Bis(aminomethyl)cyclohexane>

Bis(aminomethyl)cyclohexane is produced by hydrogenating xylylenediamine in the presence of a catalyst using, for example, at least one solvent selected from the group consisting of alkylamines and alkylenediamines.

### 1. Xylylenediamine

For xylylenediamine that is the starting material substance, three isomers, ortho, meta, and para, are present, and either a single or a mixture of these can be the starting material substance. In this embodiment, at least one selected from the group consisting of the meta isomer and the para isomer is preferably used as the starting material, and the meta isomer is more preferably used as the starting material.

### 2. Solvent

In the production of bis(aminomethyl)cyclohexane in this embodiment, at least one selected from the group consisting of alkylamines and alkylenediamines is preferably used as a solvent.

In this embodiment, the selection of a solvent that can be circularly used is preferable. For example, a solvent that can be separated by distillation from the reaction product and circularly used, or bis(aminomethyl)cyclohexane as an alkylenediamine solvent is preferably selected. An advantage of bis(aminomethyl)cyclohexane is that it need not be separated by distillation, as described later. Specifically, using at least one solvent selected from the group consisting of alkylamines and alkylenediamines having 1 to 18 carbon atoms that is liquid at normal temperature is effective in the suppression of the production of by-products and an increase in the yield of the target.

Examples of specific alkylamines can include methylamine, dimethylamine, trimethylamine, ethylamine, diethylamine, triethylamine, propylamine, dipropylamine, isopropylamine, diisopropylamine, butylamine, dibutylamine, tributylamine, hexylamine, cyclohexylamine, and 2-ethylhexylamine. Examples of specific alkylenediamines include ethylenediamine, propylenediamine, 1,4-butylenediamine, hexamethylenediamine, or bis(aminomethyl)cyclohexane.

Among these, particularly bis(aminomethyl)cyclohexane is advantageous in terms of the process because the product obtained by the hydrogenation of xylylenediamine can also be circularly used as a solvent, and solvent removal and the like are not needed in subsequent steps. The bis(aminomethyl)cyclohexane used as a solvent is preferably the same isomer as the bis(aminomethyl)cyclohexane that is the product. For example, when the product is 1,3-bis(aminomethyl)cyclohexane, the bis(aminomethyl)cyclohexane used as a solvent is preferably 1,3-bis(aminomethyl)cyclohexane.

As the solvent, at least one selected from the group consisting of alkylamines and alkylenediamines needs to be used, as described above, but the solvent may be a single solvent or a mixture. A mixture of at least one selected from the group consisting of alkylamines and alkylenediamines and another organic solvent can also be used.

As another organic solvent to be mixed, for example, alcohols such as methanol, ethanol, isopropyl alcohol, and n-propyl alcohol are used.

The weight ratio of the starting material xylylenediamine and the solvent (xylylene diamine: solvent) is preferably 1:100 to 1:1, more preferably 1:80 to 1:3, and further preferably 1:50 to 1:10. When a mixed solvent is used as the solvent, the total weight of the mixed solvent satisfies the above ranges of the weight ratio.

### 3. Catalyst

The catalyst used in the production of bis(aminomethyl)cyclohexane in this embodiment is not limited, and examples of the catalyst can include known supported and non-supported metal catalysts and Raney catalysts. Among them, catalysts containing as an active metal component at least one metal selected from the group consisting of ruthenium, rhodium, nickel, cobalt, palladium, and platinum are preferable, and among them, ruthenium-containing catalysts are preferable.

In the case of a supported catalyst, examples of the support include alumina, silica, diatomaceous earth, carbon, titania, and zirconia. The catalyst may be modified as needed, by adding at least one component selected from the group consisting of Li, Na, K, Rb, Cs, Be, Ca, Ba, Ti, Cu, Cr, Zn, Mn, Mg, Fe, Ga, Ge, Nb, Ir, Pt, Bi, Al, Si, In, Sr, Ce, and Mo.

Examples of the starting material of the ruthenium-containing catalyst include metal ruthenium, ruthenium oxide, and ruthenium hydroxide, and they are preferably used in the form of being supported on alumina, diatomaceous earth, carbon, or the like.

The amount of the ruthenium-containing catalyst supported is appropriately selected according to the type and shape of the catalyst, the type of the ruthenium starting material, the reaction temperature, the amount of hydrogen supplied, and the like. For example, when an alumina support is used, about 2% by mass of ruthenium supported on 1 mm φ to 2 mm φ crushed grain alumina can be used.

Among these, a ruthenium-supported alumina catalyst is preferable as the ruthenium-containing catalyst.

In the hydrogenation reaction, an additive may be added for the purposes of reaction promotion, yield improvement, and the like. Examples of the additive include hydroxides and alcoholates of alkali metals or alkaline earth metals, and specific examples include lithium hydroxide, sodium hydroxide, and potassium hydroxide.

### 4. Hydrogenation of Xylylenediamine

The form of the hydrogenation reaction is a fixed-bed continuous flow type which is simple.

The hydrogen pressure in the hydrogenation of xylylenediamine is preferably 0.4 MPaG or more and industrially desirably 5 to 14 MPaG. The reaction temperature is preferably 50 to 150°C, more preferably 80 to 130°C.

For example, when the catalyst in which about 2% by mass of ruthenium is supported is used, the amount of the catalyst used in the hydrogenation of xylylenediamine is preferably 0.001 to 5.0, more preferably 0.001 to 2.0, in terms of WHSV based on the amount of the starting material xylylenediamine supplied.

In the hydrogenation reaction, in terms of the productivity of bis(aminomethyl)cyclohexane, conditions such as reaction temperature and the amount of the starting material supplied are preferably selected so that the conversion rate of xylylenediamine is preferably 95 mol% or more, more preferably 98 mol% or more, and further preferably the conversion rate is substantially 100 mol%.

The separation of the target from the reaction product can be easily performed by distilling off the alkylamine, the alkylenediamine, and the organic solvent at normal pressure followed by vacuum distillation.

When the hydrogenation of xylylenediamine is continuously performed using a solvent, examples can include a method in which the dissolved gas is separated from the solution including the reaction product using a gas-liquid separator, and then the solution is once recovered in solvent recovery equipment, and the solvent is separated by distillation in the recovery equipment and circularly used, and a method in which bis(aminomethyl)cyclohexane obtained by hydrogenation is partially separated and circularly used as a solvent.

When bis(aminomethyl)cyclohexane obtained by catalytic hydrogenation by the method of this embodiment is used as a solvent, it can be directly circularly used after the dissolved gas is separated from the reaction product by a gas-liquid separator.

### <Regeneration of Catalyst>

In this embodiment, as described above, in producing bis(aminomethyl)cyclohexane, the catalyst with decreased activity due to use is subjected to regeneration treatment by bringing the catalyst into contact with a hydrogen-containing gas, and then reused in a reaction system. The catalyst regeneration treatment in this embodiment is characterized by including the step (1) of maintaining the amount of bis(aminomethyl)cyclohexane in a liquid before a step (2) at 20% by mass or less, and the step (2) of heating the catalyst to 100 to 500°C and bringing the catalyst into contact with a hydrogen-containing gas. It is simple and industrially advantageous to carry out such regeneration treatment of the catalyst in the reactor used for the hydrogenation reaction of bis(aminomethyl)cyclohexane, and therefore such regeneration treatment of the catalyst is preferable.

### Step (1)

In the step (1), the amount of bis(aminomethyl)cyclohexane in the liquid before the following step (2) is maintained at 20% by mass or less. When the amount of bis(aminomethyl)cyclohexane in the liquid before the step (2) is more than 20% by mass, the deposition of a solid on the catalyst is caused in the step (2), and the regeneration treatment of the catalyst is impossible. The adjustment of the amount of bis(aminomethyl)cyclohexane in the liquid in this step (1) can be performed, for example, by subjecting the catalyst to washing treatment using a washing liquid.

Specifically, when the amount of bis(aminomethyl)cyclohexane in the liquid before the step (2) is more than 20% by mass, bis(aminomethyl)cyclohexane in the liquid before the step (2) can be maintained at an amount defined in the step (1) by washing the catalyst using a washing liquid until the amount of bis(aminomethyl)cyclohexane in the liquid after washing reaches 20% by mass or less.

The measurement of the amount of bis(aminomethyl)cyclohexane in the liquid can be performed by a known method such as gas chromatography by partially taking out the washing liquid before and after washing.

In this embodiment, when a washing liquid is used, the amount of bis(aminomethyl)cyclohexane in the liquid is the concentration of bis(aminomethyl)cyclohexane in the washing liquid after washing, and when no washing liquid is used, the amount of bis(aminomethyl)cyclohexane in the liquid is the concentration of bis(aminomethyl)cyclohexane in the solution including the reaction product obtained from the reactor. The mass of the entire liquid including bis(aminomethyl)cyclohexane (excluding the mass of solids such as the catalyst mixed in the liquid) is 100% by mass.

A more specific description will be given.

When bis(aminomethyl)cyclohexane is used as a reaction solvent, the amount of bis(aminomethyl)cyclohexane in the liquid before the step (2) is usually more than 20% by mass, and therefore according to one aspect of this embodiment, in the step (1), the catalyst is subjected to washing treatment using a washing liquid until the amount of bis(aminomethyl)cyclohexane in the liquid reaches 20% by mass or less. When the amount of bis(aminomethyl)cyclohexane in the liquid before the step (2) is 20% by mass or less, the process may proceed to the step (2) without the washing step.

When the amount of bis(aminomethyl)cyclohexane defined in the step (1) is satisfied when a solvent other than bis(aminomethyl)cyclohexane is used as the reaction solvent in the production of bis(aminomethyl)cyclohexane, the process proceeds to the following step (2) without the washing treatment or the like being particularly performed. Also when a solvent other than bis(aminomethyl)cyclohexane is used, the amount of bis(aminomethyl)cyclohexane in the liquid can be adjusted by washing treatment with a washing liquid so as to satisfy the requirement of the step (1), as described above, when the amount of bis(aminomethyl)cyclohexane before the step (2) is more than 20% by mass.

As the washing liquid, at least one selected from the group consisting of water, aliphatic hydrocarbons, aromatic hydrocarbons, ketones, and alcohols can be used, and these may be a single or a mixture. Examples of the aliphatic hydrocarbons include n-hexane, n-heptane, and cyclohexane. Examples of the aromatic hydrocarbons include meta-xylene, para-xylene, ortho-xylene, benzene, and toluene. Examples of the ketones include acetone, methyl ethyl ketone, methyl isobutyl ketone, and cyclohexanone. Examples of the alcohols include methanol, ethanol, isopropyl alcohol, normal propyl alcohol, isobutyl alcohol, and normal butyl alcohol. Among these, at least one selected from the group consisting of water, aliphatic hydrocarbons having 5 or more and 15 or less carbon atoms, aromatic hydrocarbons having 6 or more and 15 or less carbon atoms, ketones having 3 or more and 15 or less carbon atoms, and alcohols having 1 or more and 15 or less carbon atoms is preferable, and in terms of safety and environment and cost, more preferably, the washing liquid is water alone or includes water.

The washing liquid may be at normal temperature or heated. When the washing liquid is heated, and, for example, when the washing liquid is water, it is preferably heated to about 30 to 90°C at which it does not volatilize at normal pressure.

The present inventors have detected that when the hydrogen regeneration treatment of a catalyst is performed in a bis(aminomethyl)cyclohexane production line, solid deposition occurs on the catalyst layer and a heat exchanger and the like used in subsequent steps, causing trouble in production. In the regeneration treatment of a catalyst used for the hydrogenation of dicyanobenzene as in PTL2, such a phenomenon is not observed. As a result of diligent studies, it has been found that the trouble is solved by maintaining the amount of bis(aminomethyl)cyclohexane in a liquid before bringing the catalyst into contact with a hydrogen-containing gas in the step (2) at 20% by mass or less. According to one aspect of this embodiment, the amount of bis(aminomethyl)cyclohexane in the step (1) can be adjusted by washing the catalyst using a washing liquid, as described above.

In the step (1), the amount of bis(aminomethyl)cyclohexane in the liquid before the step (2) is preferably maintained at 18% by mass or less, more preferably 15% by mass or less, and further preferably 10% by mass or less.

The form of washing is not particularly limited, and the catalyst may be washed in the reactor used for the production of bis(aminomethyl)cyclohexane, or the catalyst may be taken out from the production reactor and separately washed. The form of washing is not particularly limited. When the catalyst is washed in the production reactor, the simplification of the process can be promoted, and this is industrially advantageous and therefore preferable.

Examples of the washing method can include (i) a method of filling the catalyst layer with a washing liquid and repeating liquid removal, and (ii) a method of flowing a washing liquid through the catalyst layer. In either case of (i) and (ii) as the forms of washing, the washing liquid may be circulated in order to increase washing efficiency.

When the washing liquid is circulated, the circulation time is preferably roughly about 0.5 to 10 h though it differs depending on the amount of the catalyst. In the case of the (i) form of washing, in order to increase washing efficiency, the operation of once removing the liquid and then filling the catalyst layer with a new washing liquid is performed, and this may be repeated.

### Step (2)

In the step, the catalyst with decreased activity is regenerated by heating the catalyst to 100 to 500°C and bringing the catalyst into contact with a hydrogen-containing gas. When the treatment temperature is lower than 100°C, the effect of the regeneration treatment is not sufficient. When the treatment temperature is more than 500°C, the catalyst may deteriorate.

The regeneration treatment of the catalyst may be performed in the reactor in which bis(aminomethyl)cyclohexane is produced, or may be carried out when the catalyst is drawn from the reactor. The regeneration treatment of the catalyst is preferably performed in the reactor in which bis(aminomethyl)cyclohexane is produced, because this is simple and industrially advantageous. As described above, this reactor is a fixed-bed reactor.

In this embodiment, the regeneration treatment is performed with the catalyst temperature rise rate during contact with the hydrogen-containing gas being preferably 40°C/min or less (including zero), more preferably 30°C/min or less, and further preferably 20°C/min or less. As described above, the regeneration treatment with the hydrogen-containing gas is preferably carried out in the range of 100 to 500°C, and also when a hydrogen-containing gas and the catalyst are brought into contact with each other at less than 100°C prior to the regeneration treatment in the temperature range, the catalyst temperature rise rate is preferably controlled at 40°C/min or less. In the regeneration treatment of the catalyst with the hydrogen-containing gas, the temperature of the catalyst may rise suddenly and be uncontrollable during the treatment. The sudden rise in the temperature of the catalyst is not preferable in terms of the safety and stability of the production operation and moreover causes the deterioration of catalytic performance and therefore should be avoided.

According to the findings of the present invention and the like, the rise in the temperature of the catalyst is deeply related to the hydrogen-containing gas supply rate during the regeneration treatment. Therefore, by controlling the hydrogen-containing gas supply rate in the regeneration treatment, a sudden rise in the temperature of the catalyst can be avoided. That is, by setting the hydrogen-containing gas supply rate low, a possible sudden rise in the temperature of the catalyst can be avoided. The hydrogen-containing gas supply rate is preferably adjusted at a rate at which the catalyst temperature rise rate is 40°C/min or less. The specific hydrogen-containing gas supply rate differs depending on the type and use history of the catalyst and the temperature of the catalyst but is preferably 0.001 to 2000 NL/h (N: normal state (0°C, 0.1 MPa (1 atm))), more preferably 0.001 to 1000 NL/h, per kg of the catalyst.

The hydrogen-containing gas used for the regeneration of the catalyst may include inert impurities that do not hinder catalyst regeneration, for example, methane and nitrogen. The hydrogen pressure is preferably 0.01 kPa to 30 MPa, more preferably 0.1 kPa to 15 MPa. When the hydrogen pressure is set low (for example, 0.1 MPa or less), using an inert diluent gas such as nitrogen is simple and preferable.

In this embodiment, the regeneration step (2) with a hydrogen-containing gas is preferably performed in the following two stages. That is, the regeneration treatment of the catalyst with a hydrogen-containing gas is more preferably performed by two steps, (2-1) a step of bringing the catalyst into contact with a hydrogen-containing gas at a temperature in the range of 100 to 200°C and at an average temperature of 180°C or less, for example, for 1 h or more (hereinafter sometimes referred to as a low temperature treatment step), and (2-2) a step of further bringing the catalyst subjected to contact treatment in the step (2-1) into contact with a hydrogen-containing gas at a temperature in the range of more than 200°C to 500°C (hereinafter sometimes referred to as a high temperature treatment step). By making the regeneration treatment the two steps, the catalyst can be more sufficiently regenerated. In addition, by making the regeneration treatment two steps, heat generation and gas generation due to the regeneration treatment can be made mild, and therefore this is preferable particularly in industrial-scale apparatuses.

### (2-1) Low Temperature Treatment Step

The temperature during the catalyst regeneration in the low temperature treatment step (2-1) is in the range of 100 to 200°C, preferably 120 to 180°C, and the average temperature is 180°C or less. The average temperature refers to the time average temperature in the low temperature treatment step carried out at 100 to 200°C and is defined by a value obtained by dividing by treatment time length a value obtained by integrating temperature with time. In the low temperature treatment step (2-1), the temperature during the treatment may be changed in the range of 100 to 200°C. For example, a step in which a constant temperature holding process is combined with a temperature rise or temperature fall process is also possible.

The treatment time in the low temperature treatment step is usually 1 h or more, preferably 1 to 200 h.

In the low temperature treatment step (2-1), hydrogen is supplied so that the catalyst temperature rise rate is preferably 40°C/min or less (including zero), more preferably 30°C/min or less, and particularly preferably 20°C/min or less.

### (2-2) High Temperature Treatment Step

The temperature during the catalyst regeneration in the high temperature treatment step (2-2) is in the range of more than 200°C to 500°C, preferably 210°C to 400°C, and more preferably 220°C to 350°C. In the high temperature treatment step (2-2), the temperature during the treatment may be changed within the ranges. For example, a step in which a constant temperature holding process is combined with a temperature rise or temperature fall process is also possible.

The treatment time in the high temperature treatment step can usually be selected from the group consisting of the range of 3 to 300 h. The treatment time and the treatment temperature depend on the type of the catalyst and the extent of decreasing of activity. When the extent of decreasing of activity is intense, the treatment time is preferably long.

In the high temperature treatment step (2-2), hydrogen is preferably supplied while control is performed so that the catalyst temperature rise rate is preferably 40°C/min or less (including zero), more preferably 30°C/min or less, and particularly preferably 20°C/min or less.

As described above, the rise in the temperature of the catalyst is deeply related to the hydrogen supply rate during the regeneration treatment. Therefore, also in the low temperature treatment step (2-1) and high temperature treatment step (2-2), by controlling the hydrogen supply rate in the low temperature treatment step and the high temperature treatment step, a sudden rise in the temperature of the catalyst can be avoided.

The hydrogen supply rate is adjusted while the temperature of the catalyst is monitored so that the catalyst temperature rise rate is 40°C/min or less. The specific hydrogen supply rate differs depending on the type and use history of the catalyst and the temperature of the catalyst but is preferably 0.001 to 2000 NL/h (N: normal state (0°C, 0.1 MPa (1 atm))), more preferably 0.001 to 1000 NL/h, per kg of the catalyst.

The hydrogen-containing gases used in the low temperature treatment step (2-1) and the high temperature treatment step (2-2) may include inert impurities that do not hinder catalyst regeneration, for example, methane and nitrogen. The hydrogen pressure in the low temperature treatment step (2-1) and the high temperature treatment step (2-2) is preferably 0.01 kPa to 30 MPa, more preferably 0.1 kPa to 15 MPa. When the hydrogen pressure is set low (for example, 0.1 MPa or less), using an inert diluent gas such as nitrogen is simple and preferable. The compositions of the hydrogen-containing gases used in the low temperature treatment step (2-1) and the high temperature treatment step (2-2) may be the same or different.

By the regeneration treatment, catalyst regeneration can be carried out while the deposition of a solid on the catalyst is prevented, and the catalytic activity that is decreased due to use can be recovered. Since the regeneration treatment is performed in a fixed-bed form, the deposition of a solid on the catalyst layer can be prevented, and therefore the regeneration treatment can be performed without problems.

The activity of the catalyst is recovered by the regeneration treatment, and the catalyst can be subjected to reuse as a xylylenediamine hydrogenation reaction catalyst, and bis(aminomethyl)cyclohexane can be produced.

### Examples

The present invention will be specifically described based on the Examples shown below, but the present invention is not limited by these Examples.

### Example 1

### <Hydrogenation of Xylylenediamine>

As a catalyst, a commercially available 2% by mass ruthenium -supported alumina catalyst was used.

A fixed-bed continuous flow type tubular reactor (inner diameter 34 cm, filling height 200 cm) was filled with 130 kg of the catalyst. The catalyst was reduced and activated under a hydrogen gas flow at 260°C, and then a mixture including 5% by mass of starting material meta-xylylenediamine and 95% by mass of solvent 1,3-bis(aminomethyl)cyclohexane was supplied to the reactor under the conditions of a reaction pressure of 8 MPaG and a reaction temperature of 100°C. The amount of the starting material supplied (referring to the mixture of the starting material and the solvent) at this time was 11 kg/h.

When the obtained reactor outlet liquid was analyzed by gas chromatography, the conversion rate of meta-xylylenediamine was 99.5 mol%. When the reaction was performed for 90 days, the conversion rate of meta-xylylenediamine decreased to 98.8 mol%, and therefore the reaction was stopped.

The conversion rate of meta-xylylenediamine (hereinafter also referred to as "MXDA") was calculated by the following method:
conversion rate (mol%) = ((MXDA mass in starting material supply liquid - MXDA mass in reactor outlet liquid)/MXDA molecular weight)/(MXDA mass in starting material supply liquid/MXDA molecular weight) × 100

The conditions of the gas chromatography are as follows:
Apparatus: product name "GC-2010 plus" manufactured by SHIMADZU CORPORATION
Column: DB-1 (inner diameter 0.53 mm, length 30 m, film thickness 1.50 µm) manufactured by Agilent Technologies Japan, Ltd.
Carrier gas: He gas was flowed at a flow rate of 2.3 mL/min.
Amount of sample injected: 1.0 µL
Detector: hydrogen flame ionization detector (FID)
Detector temperature: 280°C
Vaporization chamber temperature: 280°C
Temperature rise conditions: The temperature was held at 100°C for 18 min, raised to 120°C at 5°C/min, and held for 20 min. Subsequently, the temperature was raised to 280°C at 10°C/min and held for 10 min.
Sample preparation method: 0.1 g of diphenylmethane as an internal standard substance was added to 0.1 g of a sample, and the mixture was diluted with 10 g of methanol.

### <Regeneration Treatment of Catalyst>

The pressure of the reactor was set at normal pressure, and then the catalyst layer was washed with water until the amount of 1,3-bis(aminomethyl)cyclohexane in the liquid reached 15% by mass (step (1)).

The amount of 1,3-bis(aminomethyl)cyclohexane in the liquid was quantified by gas chromatography by collecting the washing liquid. The conditions of the gas chromatography are as follows:

### (Gas Chromatography Measurement Conditions)

Apparatus: product name "GC-2010 plus" manufactured by SHIMADZU CORPORATION
Column: CP-Volamine (inner diameter 0.32 mm, length 60 m) manufactured by Agilent Technologies Japan, Ltd.
Carrier gas: He gas was flowed at a flow rate of 1.5 mL/min.
Amount of sample injected: 1.0 µL
Detector: hydrogen flame ionization detector (FID)
Detector temperature: 260°C
Vaporization chamber temperature: 230°C
Temperature rise conditions: The temperature was held at 230°C for 35 min.
Sample preparation method: 0.1 g of diphenylmethane as an internal standard substance was added to 0.1 g of a sample, and the mixture was diluted with 10 g of methanol.

Next, hydrogen gas was supplied to the catalyst layer after washing at a rate of 240 NL/h per kg of the catalyst, and heating was performed from room temperature to 150°C, and then the hydrogen flow was held at 150°C for 2 h (low temperature heating step (2-1)). Then, the temperature was raised to 260°C at a rate of 0.1°C/min, and the hydrogen flow was held at 260°C for 10 h (high temperature heating step (2-2)), and then the temperature was lowered to room temperature. In the step (2), a rise in the temperature of the catalyst at 40°C/min or more was not observed, and the catalyst temperature rise rate was 1°C/min or less.

### <Hydrogenation Reaction Using Regenerated Catalyst>

The hydrogenation reaction of xylylenediamine was performed under the same conditions as above, using the catalyst after regeneration. At a reaction temperature of 100°C, the conversion rate of meta-xylylenediamine was 99.8 mol%, showing a result substantially equivalent to that in the case where the unused catalyst was used.

### Example 2

The hydrogenation of meta-xylylenediamine was performed as in Example 1. Catalyst regeneration treatment was performed as in Example 1 except that after the hydrogenation reaction was stopped, the catalyst layer was washed with water until the amount of 1,3-bis(aminomethyl)cyclohexane in the liquid reached 1% by mass (step (1)). The catalyst after the regeneration treatment was reusable as in Example 1. Also in Example 2, a rise in the temperature of the catalyst at 40°C/min or more in the step (2) was not observed, and the catalyst temperature rise rate was 1°C/min or less.

### Example 3

The hydrogenation of meta-xylylenediamine was performed as in Example 1. Catalyst regeneration treatment was performed as in Example 1 except that after the hydrogenation reaction was stopped, the catalyst layer was washed with water until the amount of 1,3-bis(aminomethyl)cyclohexane in the liquid reached 5% by mass (step (1)). The catalyst after the regeneration treatment was reusable as in Example 1. Also in Example 3, a rise in the temperature of the catalyst at 40°C/min or more in the step (2) was not observed, and the catalyst temperature rise rate was 1°C/min or less.

### Comparative Example 1

The hydrogenation of meta-xylylenediamine was performed as in Example 1. The regeneration treatment of the catalyst in which the catalyst was brought into contact with hydrogen gas was attempted as in Example 1 except that after the hydrogenation reaction was stopped, the washing of the catalyst layer was not carried out. However, about 7 h after the supply of hydrogen gas, the flow rate of hydrogen gas decreased by about 35%. A solid product deposited on the catalyst layer, and the heat exchanger and the like used in the subsequent steps were blocked, and the catalyst regeneration treatment could not be performed.

### Comparative Example 2

The hydrogenation of meta-xylylenediamine was performed as in Example 1. The regeneration treatment of the catalyst in which the catalyst was brought into contact with hydrogen gas was attempted as in Example 1 except that after the hydrogenation reaction was stopped, the catalyst layer was washed with water until the amount of 1,3-bis(aminomethyl)cyclohexane in the liquid reached 50% by mass. However, about 11 h after the supply of hydrogen gas, the flow rate of hydrogen gas decreased by about 35%. A solid product deposited on the catalyst layer, and the heat exchanger and the like used in the subsequent steps were blocked, and the catalyst regeneration treatment could not be performed.

### Comparative Example 3

The hydrogenation of meta-xylylenediamine was performed as in Example 1. The regeneration treatment of the catalyst in which the catalyst was brought into contact with hydrogen gas was attempted as in Example 1 except that after the hydrogenation reaction was stopped, the catalyst layer was washed with water until the amount of 1,3-bis(aminomethyl)cyclohexane in the liquid reached 30% by mass. However, about 13 h after the supply of hydrogen gas, the flow rate of hydrogen gas decreased by about 35%. A solid product deposited on the catalyst layer, and the heat exchanger and the like used in the subsequent steps were blocked, and the catalyst regeneration treatment could not be performed.

### Example 4

As a catalyst, a 2% by mass ruthenium-supported alumina catalyst was used.

A tubular reactor (inner diameter 34 cm, filling height 200 cm) was filled with 0.14 m³ of the catalyst. The catalyst was reduced and activated under a hydrogen gas flow at 260°C, and then a mixture including 5% by mass of starting material meta-xylylenediamine and 95% by mass of solvent 1,3-bis(aminomethyl)cyclohexane was supplied to the reactor under the conditions of a reaction pressure of 8 MPaG and a reaction temperature of 100°C. The amount of the starting material supplied (referring to the mixture of the starting material and the solvent) at this time was 11 kg/h.

After the reaction was performed for 90 days, the reaction was stopped. The pressure of the reactor was set at normal pressure, and then the catalyst layer was washed with methanol until the amount of 1,3-bis(aminomethyl)cyclohexane in the liquid reached 15% by mass (step (1)).

Hydrogen gas was supplied at a rate of 240 NL/h per kg of the catalyst, and heating was performed from room temperature to 150°C, and then the hydrogen flow was held at 150°C for 2 h (low temperature heating step (2-1)). Then, the temperature was raised to 260°C at a rate of 0.1°C/min, and the hydrogen flow was held at 260°C for 10 h (high temperature heating step (2-2)), and then the temperature was lowered to room temperature. The catalyst after the regeneration treatment was reusable as in Example 1. In the step (2), a rise in the temperature of the catalyst at 40°C/min or more was not observed, and the catalyst temperature rise rate was 1°C/min or less.

### Comparative Example 4

The hydrogenation of meta-xylylenediamine was performed as in Example 4. The regeneration treatment of the catalyst in which the catalyst was brought into contact with hydrogen gas was attempted as in Example 4 except that after the hydrogenation reaction was stopped, the catalyst layer was washed with methanol until the amount of 1,3-bis(aminomethyl)cyclohexane in the liquid reached 50% by mass. However, about 12 h after the supply of hydrogen gas, the flow rate of hydrogen gas decreased by about 35%. A solid product deposited on the catalyst layer, and the heat exchanger and the like used in the subsequent steps were blocked, and the catalyst regeneration treatment could not be performed.

### Comparative Example 5

The hydrogenation of meta-xylylenediamine was performed as in Example 4. The regeneration treatment of the catalyst in which the catalyst was brought into contact with hydrogen gas was attempted as in Example 4 except that after the hydrogenation reaction was stopped, the catalyst layer was washed with methanol until the amount of 1,3-bis(aminomethyl)cyclohexane in the liquid reached 30% by mass. However, about 18 h after the supply of hydrogen gas, the flow rate of hydrogen gas decreased by about 35%. A solid product deposited on the catalyst layer, and the heat exchanger and the like used in the subsequent steps were blocked, and the catalyst regeneration treatment could not be performed.

### Example 5

As a catalyst, a 2% by mass ruthenium-supported alumina catalyst was used.

A tubular reactor (inner diameter 34 cm, filling height 200 cm) was filled with 0.14 m³ of the catalyst. The catalyst was reduced and activated under a hydrogen gas flow at 260°C, and then a mixture including 5% by mass of starting material meta-xylylenediamine and 95% by mass of solvent 1,3-bis(aminomethyl)cyclohexane was supplied to the reactor under the conditions of a reaction pressure of 8 MPaG and a reaction temperature of 100°C. The amount of the starting material supplied (referring to the mixture of the starting material and the solvent) at this time was 11 kg/h.

After the reaction was performed for 90 days, the reaction was stopped. The pressure of the reactor was set at normal pressure, and then the catalyst layer was washed with meta-xylene until the amount of 1,3-bis(aminomethyl)cyclohexane in the liquid reached 15% by mass (step (1)).

Hydrogen gas was supplied at a rate of 240 NL/h per kg of the catalyst, and heating was performed from room temperature to 150°C, and then the hydrogen flow was held at 150°C for 2 h (low temperature heating step (2-1)). Then, the temperature was raised to 260°C at a rate of 0.1°C/min, and the hydrogen flow was held at 260°C for 10 h (high temperature heating step (2-2)), and then the temperature was lowered to room temperature. The catalyst after the regeneration treatment was reusable as in Example 1. In the step (2), a rise in the temperature of the catalyst at 40°C/min or more was not observed, and the catalyst temperature rise rate was 1°C/min or less.

### Comparative Example 6

The hydrogenation of meta-xylylenediamine was performed as in Example 5. The regeneration treatment of the catalyst in which the catalyst was brought into contact with hydrogen gas was attempted as in Example 5 except that after the hydrogenation reaction was stopped, the catalyst layer was washed with meta-xylene until the amount of 1,3-bis(aminomethyl)cyclohexane in the liquid reached 30% by mass. However, about 23 h after the supply of hydrogen gas, the flow rate of hydrogen gas decreased by about 35%. A solid product deposited on the catalyst layer, and the heat exchanger and the like used in the subsequent steps were blocked, and the catalyst regeneration treatment could not be performed.

### Example 6

As a catalyst, a 2% by mass ruthenium-supported alumina catalyst was used.

A tubular reactor (inner diameter 34 cm, filling height 200 cm) was filled with 0.14 m³ of the catalyst. The catalyst was reduced and activated under a hydrogen gas flow at 260°C, and then a mixture including 5% by mass of starting material meta-xylylenediamine and 95% by mass of solvent 1,3-bis(aminomethyl)cyclohexane was supplied to the reactor under the conditions of a reaction pressure of 8 MPaG and a reaction temperature of 100°C. The amount of the starting material supplied (referring to the mixture of the starting material and the solvent) at this time was 11 kg/h.

After the reaction was performed for 90 days, the reaction was stopped. The pressure of the reactor was set at normal pressure, and then the catalyst layer was washed with acetone until the amount of 1,3-bis(aminomethyl)cyclohexane in the liquid reached 15% by mass (step (1)).

Hydrogen gas was supplied at a rate of 240 NL/h per kg of the catalyst, and heating was performed from room temperature to 150°C, and then the hydrogen flow was held at 150°C for 2 h (low temperature heating step (2-1)). Then, the temperature was raised to 260°C at a rate of 0.1°C/min, and the hydrogen flow was held at 260°C for 10 h (high temperature heating step (2-2)), and then the temperature was lowered to room temperature. The catalyst after the regeneration treatment was reusable as in Example 1. In the step (2), a rise in the temperature of the catalyst at 40°C/min or more was not observed, and the catalyst temperature rise rate was 1°C/min or less.

### Comparative Example 7

The hydrogenation of meta-xylylenediamine was performed as in Example 6. The regeneration treatment of the catalyst in which the catalyst was brought into contact with hydrogen gas was attempted as in Example 6 except that after the hydrogenation reaction was stopped, the catalyst layer was washed with acetone until the amount of 1,3-bis(aminomethyl)cyclohexane in the liquid reached 30% by mass. However, about 19 h after the supply of hydrogen gas, the flow rate of hydrogen gas decreased by about 35%. A solid product deposited on the catalyst layer, and the heat exchanger and the like used in the subsequent steps were blocked, and the catalyst regeneration treatment could not be performed.

### Example 7

As a catalyst, a 2% by mass ruthenium-supported alumina catalyst was used.

A tubular reactor (inner diameter 34 cm, filling height 200 cm) was filled with 0.14 m³ of the catalyst. The catalyst was reduced and activated under a hydrogen gas flow at 260°C, and then a mixture including 5% by mass of starting material meta-xylylenediamine and 95% by mass of solvent 1,3-bis(aminomethyl)cyclohexane was supplied to the reactor under the conditions of a reaction pressure of 8 MPaG and a reaction temperature of 100°C. The amount of the starting material supplied (referring to the mixture of the starting material and the solvent) at this time was 11 kg/h.

After the reaction was performed for 90 days, the reaction was stopped. The pressure of the reactor was set at normal pressure, and then the catalyst layer was washed with n-heptane until the amount of 1,3-bis(aminomethyl)cyclohexane in the liquid reached 15% by mass (step (1)).

Hydrogen gas was supplied at a rate of 240 NL/h per kg of the catalyst, and heating was performed from room temperature to 150°C, and then the hydrogen flow was held at 150°C for 2 h (low temperature heating step (2-1)). Then, the temperature was raised to 260°C at a rate of 0.1°C/min, and the hydrogen flow was held at 260°C for 10 h (high temperature heating step (2-2)), and then the temperature was lowered to room temperature. The catalyst after the regeneration treatment was reusable as in Example 1. In the step (2), a rise in the temperature of the catalyst at 40°C/min or more was not observed, and the catalyst temperature rise rate was 1°C/min or less.

### Comparative Example 8

The hydrogenation of meta-xylylenediamine was performed as in Example 7. The regeneration treatment of the catalyst in which the catalyst was brought into contact with hydrogen gas was attempted as in Example 7 except that after the hydrogenation reaction was stopped, the catalyst layer was washed with heptane until the amount of 1,3-bis(aminomethyl)cyclohexane in the liquid reached 30% by mass. However, about 18 h after the supply of hydrogen gas, the flow rate of hydrogen gas decreased by about 35%. A solid product deposited on the catalyst layer, and the heat exchanger and the like used in the subsequent steps were blocked, and the catalyst regeneration treatment could not be performed.

## Claims

1. A method for producing bis(aminomethyl)cyclohexane, comprising hydrogenating xylylenediamine in the presence of a solvent and a catalyst in a fixed-bed continuous flow type reactor, wherein the catalyst with decreased activity due to use is treated in said reactor in a catalyst regeneration treatment step comprising the following step (1) and step (2), and then reused in a reaction system:
step (1): maintaining an amount of bis(aminomethyl)cyclohexane in a liquid before the step (2) at 20% by mass or less,
step (2): heating the catalyst to 100 to 500°C and bringing the catalyst into contact with a hydrogen-containing gas.

2. The method for producing bis(aminomethyl)cyclohexane according to claim 1, wherein the solvent comprises at least one selected from the group consisting of alkylamines and alkylenediamines.

3. The method for producing bis(aminomethyl)cyclohexane according to claim 1 or 2, wherein the amount of bis(aminomethyl)cyclohexane in the liquid before the step (2) is maintained at 20% by mass or less by subjecting the catalyst to washing treatment using a washing liquid.

4. The method for producing bis(aminomethyl)cyclohexane according to claim 3, wherein the washing liquid comprises at least one selected from the group consisting of water, aliphatic hydrocarbons, aromatic hydrocarbons, ketones, and alcohols.

5. The method for producing bis(aminomethyl)cyclohexane according to any one of claims 1 to 4, wherein the step (2) comprise the following steps (2-1) and (2-2):
(2-1) a step of bringing the catalyst into contact with a hydrogen-containing gas at a temperature in the range of 100 to 200°C and at an average temperature of 180°C or less,
(2-2) a step of further bringing the catalyst subjected to contact treatment in the step (2-1) into contact with a hydrogen-containing gas at a temperature in the range of more than 200°C to 500°C.

6. The method for producing bis(aminomethyl)cyclohexane according to any one of claims 1 to 5, wherein the contact with the hydrogen-containing gas in the step (2) is performed under conditions in which a catalyst temperature rise rate is 40°C/min or less.

7. The method for producing bis(aminomethyl)cyclohexane according to any one of claims 1 to 6, wherein in the step (2), a hydrogen-containing gas supply rate is controlled so that the catalyst temperature rise rate is 40°C/min or less.

8. The method for producing bis(aminomethyl)cyclohexane according to any one of claims 1 to 7, wherein the hydrogen-containing gas supply rate in the step (2) is 0.001 to 1000 L/h per kg of the catalyst in a normal state at 0°C and 0.1 MPa (1 atm).

9. The method for producing bis(aminomethyl)cyclohexane according to any one of claims 1 to 8, wherein the catalyst comprises at least one selected from the group consisting of ruthenium-containing catalysts, rhodium-containing catalysts, nickel-containing catalysts, palladium-containing catalysts, and platinum-containing catalysts.

10. The method for producing bis(aminomethyl)cyclohexane according to any one of claims 1 to 9, wherein the catalyst is a ruthenium-containing catalyst.

## Patentansprüche

1. Verfahren zur Herstellung von Bis(aminomethyl)cyclohexan, umfassend die Hydrierung von Xylylendiamin in Gegenwart eines Lösungsmittels und eines Katalysators in einem Reaktor vom Festbett-Durchflusstyp, wobei der Katalysator mit einer durch Verbrauch verringerte Aktivität in dem Reaktor in einem Katalysatorregenerationsbehandlungsschritt behandelt wird, der den folgenden Schritt (1) und Schritt (2) umfasst, und dann in einem Reaktionssystem wiederverwendet wird:
Schritt (1): Aufrechterhalten einer Menge an Bis(aminomethyl)cyclohexan in einer Flüssigkeit vor Schritt (2) bei 20 Masse-% oder weniger,
Schritt (2): Erwärmen des Katalysators auf 100 bis 500°C und Inkontaktbringen des Katalysators mit einem wasserstoffhaltigen Gas.

2. Verfahren zur Herstellung von Bis(aminomethyl)cyclohexan gemäß Anspruch 1, wobei das Lösungsmittel mindestens eines, ausgewählt aus der Gruppe, bestehend aus Alkylaminen und Alkylendiaminen, umfasst.

3. Verfahren zur Herstellung von Bis(aminomethyl)cyclohexan gemäß Anspruch 1 oder 2, wobei die Menge an Bis(aminomethyl)cyclohexan in der Flüssigkeit vor Schritt (2) bei 20 Masse-% oder weniger aufrechterhalten wird, indem der Katalysator einer Waschbehandlung unter Verwendung einer Waschflüssigkeit unterzogen wird.

4. Verfahren zur Herstellung von Bis(aminomethyl)cyclohexan gemäß Anspruch 3, wobei die Waschflüssigkeit mindestens eines, ausgewählt aus der Gruppe, bestehend aus Wasser, aliphatischen Kohlenwasserstoffen, aromatischen Kohlenwasserstoffen, Ketonen und Alkoholen, umfasst.

5. Verfahren zur Herstellung von Bis(aminomethyl)cyclohexan gemäß einem der Ansprüche 1 bis 4, wobei der Schritt (2) die folgenden Schritte (2-1) und (2-2) umfasst:
(2-1) einen Schritt zum Inkontaktbringen des Katalysators mit einem wasserstoffhaltigen Gas bei einer Temperatur im Bereich von 100 bis 200°C und bei einer durchschnittlichen Temperatur von 180°C oder weniger,
(2-2) einen Schritt zum weiteren Inkontaktbringen des Katalysators, der in Schritt (2-1) einer Kontaktbehandlung unterzogen wurde, mit einem wasserstoffhaltigen Gas bei einer Temperatur im Bereich von mehr als 200°C bis 500°C.

6. Verfahren zur Herstellung von Bis(aminomethyl)cyclohexan gemäß einem der Ansprüche 1 bis 5, wobei der Kontakt mit dem wasserstoffhaltigen Gas in Schritt (2) unter Bedingungen durchgeführt wird, unter denen eine Katalysatortemperaturanstiegsgeschwindigkeit 50°C/min oder weniger beträgt.

7. Verfahren zur Herstellung von Bis(aminomethyl)cyclohexan gemäß einem der Ansprüche 1 bis 6, wobei in Schritt (2) eine Zuführgeschwindigkeit des wasserstoffhaltigen Gases so reguliert wird, dass die Katalysatortemperaturanstiegsgeschwindigkeit 40°C/min oder weniger beträgt.

8. Verfahren zur Herstellung von Bis(aminomethyl)cyclohexan gemäß einem der Ansprüche 1 bis 7, wobei die Zuführgeschwindigkeit des wasserstoffhaltigen Gases in Schritt (2) 0,001 bis 1.000 L/h pro kg des Katalysators in einem Normalzustand bei 0°C und 0,1 MPa (1 atm) beträgt.

9. Verfahren zur Herstellung von Bis(aminomethyl)cyclohexan gemäß einem der Ansprüche 1 bis 8, wobei der Katalysator mindestens einen, ausgewählt aus der Gruppe, bestehend aus rutheniumhaltigen Katalysatoren, rhodiumhaltigen Katalysatoren, nickelhaltigen Katalysatoren, palladiumhaltigen Katalysatoren und platinhaltigen Katalysatoren, umfasst.

10. Verfahren zur Herstellung von Bis(aminomethyl)cyclohexan gemäß einem der Ansprüche 1 bis 9, wobei der Katalysator ein rutheniumhaltiger Katalysator ist.

## Revendications

1. Procédé de production de bis(aminométhyl)cyclohexane, comprenant l'hydrogénation de xylylènediamine en présence d'un solvant et d'un catalyseur dans un réacteur du type à flux continu à lit fixe, dans lequel le catalyseur présentant une activité réduite en raison de son utilisation est traité dans ledit réacteur dans une étape de traitement de régénération de catalyseur comprenant l'étape (1) et l'étape (2) suivantes, puis réutilisé dans un système réactionnel :
étape (1) : maintenir une quantité de bis(aminométhyl)cyclohexane dans un liquide avant l'étape (2) à 20 % en masse ou moins,
étape (2) : chauffer le catalyseur entre 100 et 500 °C et mettre le catalyseur en contact avec un gaz contenant de l'hydrogène.

2. Procédé de production de bis(aminométhyl)cyclohexane selon la revendication 1, dans lequel le solvant comprend au moins l'un choisi dans le groupe constitué par alkylamines et alkylènediamines.

3. Procédé de production de bis(aminométhyl)cyclohexane selon la revendication 1 ou 2, dans lequel la quantité de bis(aminométhyl)cyclohexane dans le liquide avant l'étape (2) est maintenue à 20 % en masse ou moins en soumettant le catalyseur à un traitement de lavage à l'aide d'un liquide de lavage.

4. Procédé de production de bis(aminométhyl)cyclohexane selon la revendication 3, dans lequel le liquide de lavage comprend au moins l'un choisi dans le groupe constitué d'eau, hydrocarbures aliphatiques, hydrocarbures aromatiques, cétones, et alcools.

5. Procédé de production de bis(aminométhyl)cyclohexane selon l'une quelconque des revendications 1 à 4, dans lequel l'étape (2) comprend les étapes (2-1) et (2-2) suivantes :
(2-1) une étape consistant à mettre le catalyseur en contact avec un gaz contenant de l'hydrogène à une température comprise entre 100 et 200 °C et à une température moyenne de 180 °C ou moins,
(2-2) une étape consistant à mettre en outre le catalyseur soumis au traitement de contact à l'étape (2-1) en contact avec un gaz contenant de l'hydrogène à une température comprise entre plus de 200 °C et 500 °C.

6. Procédé de production de bis(aminométhyl)cyclohexane selon l'une quelconque des revendications 1 à 5, dans lequel le contact avec le gaz contenant de l'hydrogène à l'étape (2) est effectué dans des conditions dans lesquelles la vitesse d'augmentation de la température du catalyseur est de 40 °C/min ou moins.

7. Procédé de production de bis(aminométhyl)cyclohexane selon l'une quelconque des revendications 1 à 6, dans lequel, à l'étape (2), un débit d'alimentation en gaz contenant de l'hydrogène est contrôlé de sorte que la vitesse d'augmentation de la température du catalyseur soit de 40 °C/min ou moins.

8. Procédé de production de bis(aminométhyl)cyclohexane selon l'une quelconque des revendications 1 à 7, dans lequel le débit d'alimentation en gaz contenant de l'hydrogène à l'étape (2) est de 0,001 à 1 000 L/h par kg du catalyseur dans un état normal à 0 °C et 0,1 MPa (1 atm).

9. Procédé de production de bis(aminométhyl)cyclohexane selon l'une quelconque des revendications 1 à 8, dans lequel le catalyseur comprend au moins l'un choisi dans le groupe constitué de catalyseurs contenant du ruthénium, catalyseurs contenant du rhodium, catalyseurs contenant du nickel, catalyseurs contenant du palladium, et catalyseurs contenant du platine.

10. Procédé de production de bis(aminométhyl)cyclohexane selon l'une quelconque des revendications 1 à 9, dans lequel le catalyseur est un catalyseur contenant du ruthénium.
